# EUROPEAN PATENT SPECIFICATION

(11) **EP 3 251 640 B1**
(45) Date of publication and mention of the grant of the patent: **12.12.2018**
(21) Application number: 16382240.6
(22) Date of filing: 30.05.2016
(51) Int. Cl.: A61F 13/06

(54) **AN INTEGRAL PROTECTOR FOR A LIVING BEING MEMBER**
INTEGRALE SCHUTZVORRICHTUNG FÜR EIN LEBEWESEN
PROTECTION INTÉGRALE POUR UN ÉLÉMENT DE CORPS D'UN ÊTRE VIVANT

(43) Date of publication of application: 06.12.2017
(73) Proprietor: Fundació Eurecat, 08290 Cerdanyola del Vallès (ES); Fundacio Salut del Consorci Sanitari del Maresme, 08304 Mataro (ES)
(72) Inventor: ALÓS VILLACROSA, Jesús, 08301 Mataró (Barcelona) (ES); PERA ISERN, Francesc, 08370 Calella (ES); VILÀ ALFONSO, Xavier, 08360 Canet de Mar (ES); PLANAGUMÀ VILÀ, Jordi, 08302 Mataró (ES); PÉREZ SAEZ, Carles, 08304 Mataró (ES); ROIG BARDINA, Daniel, 08360 Canet de Mar (ES); ROSELL PEREA, Jaume, 08360 Canet de Mar (ES); SOLER LUQUE, Miquel, 08360 Canet de Mar (ES); GARCIA CANDEL, Virginia, 08130 Sta. Perpètua de la Mogoda (ES)
(74) Representative: Juncosa Miró, Jaime

(56) References cited:
- US-A- 4 150 442
- US-A1- 2006 155 226
- US-B1- 8 469 913

## Description

### FIELD OF THE INVENTION

The invention pertains to field of the devices for protecting by cushioning the skin of a patient in particular against foot and heel pressure ulcers that are a major healthcare problem.

Prevalence of foot ulcers among elderly people admitted to general hospitals or nursing homes is 28-35% and could be prevented by this protector. This will improve the health status and the quality of living of these frail patients and reduce healthcare expenses.

Foot and heel pressure ulcers are a major healthcare problem. A pressure ulcer is an injury to the skin, usually over a bony prominence, as a result of constant pressure due to impaired mobility (because of wheelchair-bound or bed-bound). The pressure results in reduced blood flow and eventually may cause cell death, skin breakdown, and the development of an open wound. Common sites include the sacrum (tailbone), lower back, back of the head, elbow or heels. The heel is the second most common anatomical location for pressure ulcers after sacrum. Prevalence of heel pressure ulcers has been estimated between 18 and 28% among adult population in acute care, long-term care and homecare settings. Healing times for pressure ulcers are long, in many cases over a year. If the conditions leading to the pressure sore are not rapidly corrected, the localized skin damage will spread to deeper tissue layers affecting muscle, tendon, and bone. If not adequately treated, open ulcers can become a source of pain, disability, and infection. Moreover, amputation due to persisting infection or non-healing heel ulcers may occur in up to 40% of cases.

The cause of the pressure ulcer is the reduction of the blood flow in the point of pressure which carries death cell and ulceration. The heel is at increased risk of ulceration as a result of its posterior prominence and lack of padding over the calcaneus. The hyperaemic response to pressure loading on the heel does not differ from other tissue. However, with ageing the number of capillaries is reduced, the amount of soft-tissue padding over the calcaneus decreases and blood flow at rest to the heel is relatively low. Blood flow to the heel comes through the peroneal and posterior tibial arteries, which are often affected by peripheral arterial disease. Due to the unique anatomic characteristics of the heel and its impaired ability to reperfuse, the heel is a common site for deep tissue injury. Apart from immobilization and bed confinement, main risk factors for pressure ulcers include:
a) diseases impairing sensation such as diabetic neuropathy, stroke, and nerve block after surgery or analgesia,
b) conditions that reduce blood flow to the leg such as peripheral vascular disease (with ankle-brachial indices lower than 0.8) or vasopresive medications,
c) respiratory diseases that reduce tissue oxygenation,
d) lower limb weakness due to hip fracture or total knee replacement,
e) malnutrition and low serum albumin levels,
f) diabetes, and
g) ageing.

In addition, secondary (sometimes iatrogenic) foot ulcers can occur as a consequence of mishandling of heel ulcers

### BACKGROUND OF THE INVENTION

Prevention of pressure ulcers is a key issue because treatment can be difficult and established ulcers can result in devastating consequences such as amputation or even death. Prevention of pressure ulcers is largely based upon the principle of offloading and ensuring no skin areas exposed to pressure and shear forces. All bedridden patients need to be considered at risk of pressure ulcers and must follow universal precautions including: daily inspection of feet and heels and skin integrity, assessment of quality of pulses in the foot and ankle, maintain the skin clean and moisturized by applying cream/lotion every day, float heel from the bed, prevent friction injury by applying film/foam dressings, frequent careful changing of body position (turning bedridden patients every two hours to remove pressure), get patient out of bed as soon as possible or two times a day is possible, use of special mattresses or local protection supports, use of protectors, motion exercises, management of other contributing illnesses, or implementation of a healthy diet. Relieving or reducing the pressure on the area is essential. Offloading devices should completely lift the heel from the bed. The use of pillows or boots is common. Foam mattresses are able to reduce pressure ulcer incidence. Pillows can be used in patients who are not moving their legs, but boots are needed for patients who move about in bed or who are at high risk for ulceration. Several offloading boots have showed effectiveness in reducing pressure ulcers incidence but none of them have demonstrated to be superior respect the others.

It is considered essential to decrease the pressure in the main risk areas of the foot to prevent pressure ulcers. Thus, to "float" the foot by placing a pillow under the calves or using boots or other devices that lift the heel from the bed is considered an indispensable preventing measure. However, too often the pillow move and is not located where it is needed to be. Although protecting foot devices move less than pillows, sometimes protecting devices are not well fastened and also move. It is not rare to observe these devices in a wrong position or place so they cease to have any effect. It's very important to guarantee that the device remain in place while the patient is moving the leg to avoid shear forces. On the other hand, some devices offer only partial protection, protecting only the heel and/or the ankle, but leaving other areas unprotected and exposed to pressure, friction and shear. Even more, some of these devices may incur insignificant iatrogenic ulcers due to the pressure exerted on the back of the foot or may incur in other complications such as deep vein thrombosis or Achilles tendon injury.

Many devices for the treatment of ulcers are known.

GB 2465350 discloses a generally tubular sock having a retaining means such as a pocket for connecting a support member to the sock, said support member being of a size sufficient to extend between the rear portion of the lower leg and a surface on which the subject is lying such that the heel of the subject is held above and out of contact with the surface.

DE 202005009506U1 refers to a foot bandage for preventing pressure sores in patients sleeping on side or back and includes a continuous elastic woven fabric with incorporated foam or gel cushion.

WO 2009015418 discloses a skin protector for a joint region comprising a fabric sleeve having and end portion and an opposite end portion joined by an intermediate portion which contains a bend whereby a waterproof cushioning pad or disc may be provided containing silicone gel or other suitable materials attached to said sleeve.

On the other hand US 7465283 describes a cast or support assembly comprising a compressible padding layer including a padding material formed of double knit type fabric material having an inner layer of finely woven or knit material with openings defining a smooth surface structure on the side to face the skin, and an outer layer of coarsely woven or knit material with openings therethrough spaced from said inner layer by an open work matrix of filaments which are integrally woven or knit into said inner and said outer layers of woven or knit material, the outer layer openings being larger than the openings of the inner layer, the padding layer being devoid of a hardening material such that the padding layer remains compressible upon exposure to water. Additional casting fabric is also provided, with this casting fabric being impregnated with water hardenable material.

A similar solution to the last referred patent is used in US 6981955 relating to a castliner for a patient and particularly to a sleeve-like knitted structure, for use as a castliner having superior cushioning and enhanced comfortable conformance to the body.

US 8790297 discloses an orthopaedic padding including a knitted elongate spacer fabric defining a first and second opposing faces each having pillar inlay stich construction interconnected and separated from one another through an intermediate spacer area having a needle V stich construction, wherein the orthopaedic padding is an undercast liner.

Also EP 1082075 relates to a hardenable orthopaedic support comprising a double layer fabric in the form of a flat sheet which was impregnated with a water-hardenable material and having spaced interwoven layers and an open work matrix of filaments of fibres interconnecting said layers.

The present invention aims a new innovative concept of an integral protector, reusable, for a patient member, in particular for a foot, providing a total protection of all the potential foot areas at risk against pressure ulcers (heel, back, sides) based on the principle of offloading or cushioning and ensuring no skin areas exposed to pressure and shear forces.

### BRIEF SUMMARY OF THE INVENTION

In order to solve the above mentioned objective, i.e. to improve the prevention of heel pressure ulcers and overcome the limitations of current solutions, the invention proposes developing a one-piece boot/stocking for integral foot protection in which a 3D fabrics as padding or cushioning material is used surrounding an inner textile tubular envelope of the member intended to be in contact with the skin, the assembly being able to successfully overcome functional limitations associated with current models of heel protectors on the market.

3D spacer fabric technology fits with the main drawbacks in that has to be overcome with the integral protector proposed in terms of relieving or reducing the pressure on the foot area. Textile technology will also allow the design of a product easy to use, comfortable and well fastened avoiding the exposure to friction and shear.

As known 3D fabric is a textile structure made of 2 fabrics (A and B) joined together through a monofilament which supports the 3D structure and provides higher or lower rigidity. Further, the distance between A and B fabrics can be adjusted depending on the final application. Fabrics can be subject to specific finishes in order to enhance its performance in contact with skin and the comfort of the user.

The integral foot protector of the invention wants to be a new health device to be worn in the foot to prevent pressure ulcers in high risk patients.

According to the invention the integral protector against foot and heel pressure ulcers of a patient comprises a one-piece tubular assembly consisting of:
- a textile tubular stockinette acting as a tubular envelope in contact with the skin of the member, said textile stockinette being either knitted, woven or braided, said textile stockinette comprising at least an elastic longitudinal portion, provided when knitting said stockinette and extending along a whole generatrix thereof, and
- a padding in the form of a tubular jacket made of a 3D spacer fabric providing an external sleeve, said tubular jacket receiving placed therein said textile stockinette arranged coaxial and covering an inner surface thereof, the textile stockinette being attached to said tubular jacket so that it can not move relative thereto.

Thereby the tubular jacket operates by decreasing the pressure acting on the cited living being member when in rest against a surface.

In an embodiment the referred elastic longitudinal portion of the textile tubular stockinette is a strip of regular width.

According to a preferred embodiment the inner textile tubular stockinette and the tubular envelope are joined at least by means of a seam of the mouthpiece area of both elements the cited seam of the mouthpiece being preferably done on an external peripheral zone of the tubular jacket in order not to affect the skin of a patient.

The tubular jacket can be obtained from a flat piece with two opposite cut-out portions by joining two opposite major sides thereof, said cut-out portions providing an angled or bent portion corresponding to an elbow of the tubular jacket suitable to accommodate against the external surface of a foot.

The cited angled or bent portion is obtained by means of seam of the edges of said cut-out portion in a middle region of the tubular jacket forming an angle with the axis thereof

In an embodiment the textile stockinette is further attached to said tubular jacket by means of said elastic longitudinal portion being stitched to the facing major edges of the cited flat piece forming the tubular jacket

To facilitate insertion of the foot the tubular assembly has an end portion wider than the opposite end. Further one of the ends of the tubular assembly includes a mark providing an indication of the introduction area of the member to protect.

In order to provide a reusable assembly both the textile stockinette fabric and the 3D spacer fabric are washable and breathable.

### BRIEF DESCRIPTION OF THE DRAWINGS

Fig. 1 is a perspective view of the flat 3D spacer fabric intended to provide an external sleeve or tubular jacket by joining the two major edges thereof, and with two opposite cut-out portions that then provide a bent portion.
Fig. 2 is a perspective view of the textile tubular stockinette including an elastic longitudinal portion along the whole generatrix thereof.
Fig. 3 is a perspective view of the 3D spacer fabric with the cut-out portions sewn forming an angle o with the axis thereof, providing an elbow portion.
Fig. 4 is a perspective view of the textile tubular stockinette positioned for assembly.
Fig. 5 is a perspective view of the integral protector of the invention with the textile stockinette attached to the external 3D spacer fabric sleeve.
Figs. 6 to 10 illustrate several spacer textiles known in the art that have been tested in order to find a best 3D fabric for the production of the tubular jacket or the integral protector of this invention, using fabrics with following weights and thicknesses:

| | | | |
|---|---|---|---|
| Fig. 6: Spacer fabric 1 | Weight 330 g/m² | Thickness 3,5 | mm |
| Fig. 7: Spacer fabric 2 | Weight 220 g/m² | Thickness 6 | mm |
| Fig. 8: Spacer fabric 3 | Weight 230 g/m² | Thickness 4,2 | mm |
| Fig. 9: Spacer fabric 4 | Weight 270 g/m² | Thickness 3,2 | mm |
| Fig. 10: Spacer fabric 5 | Weight 310 g/m² | Thickness 3,5 | mm |

### DETAILED DESCRIPTION OF THE INVENTION

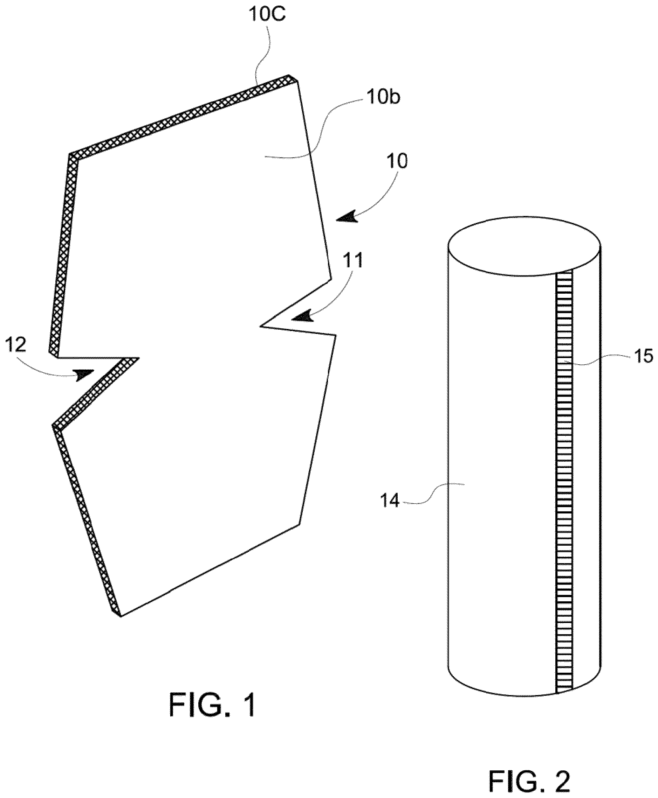

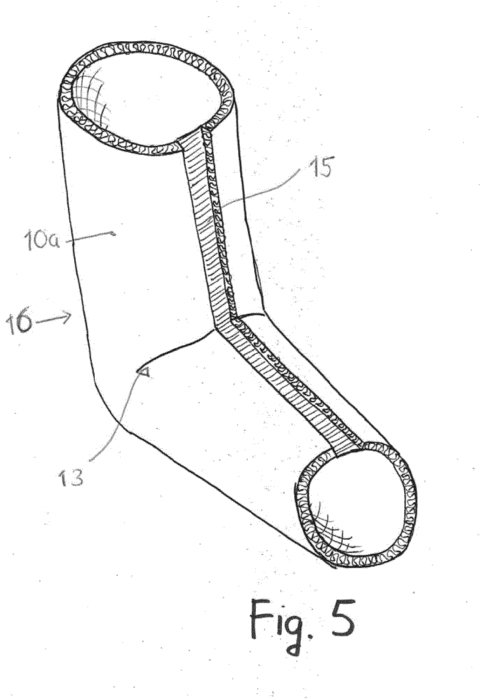

Fig. 1 details a flat piece of a 3D-spacer fabric 10 including a double layer fabric 10a, 10b with an open-work of filaments or fibres 10c interconnecting said layers 10a, 10b, The flat piece of 3D-spacer fabric 10 includes along two opposite major edges two opposite cut out portions 11, 12, that when these major edges and the edges of the cut out portions 11, 12 are joined (see Fig. 5) by stitching o sewing provide a bent part 13 conforming an elbow.

Fig. 2 illustrates a textile tubular stockinette 14 which makes the interior part of the tubular assembly constituting the proposed integral protector. In the figure it has been indicated an elastic longitudinal portion 15, extending along a whole generatrix of the stockinette 14, provided while knitting the stockinette, in the form of a strip of regular width. This elastic strip provides a means allowing that the stockinette acts as a tubular envelope tightly overlapped on the section of the member to be protected, for example a joint region of a foot.

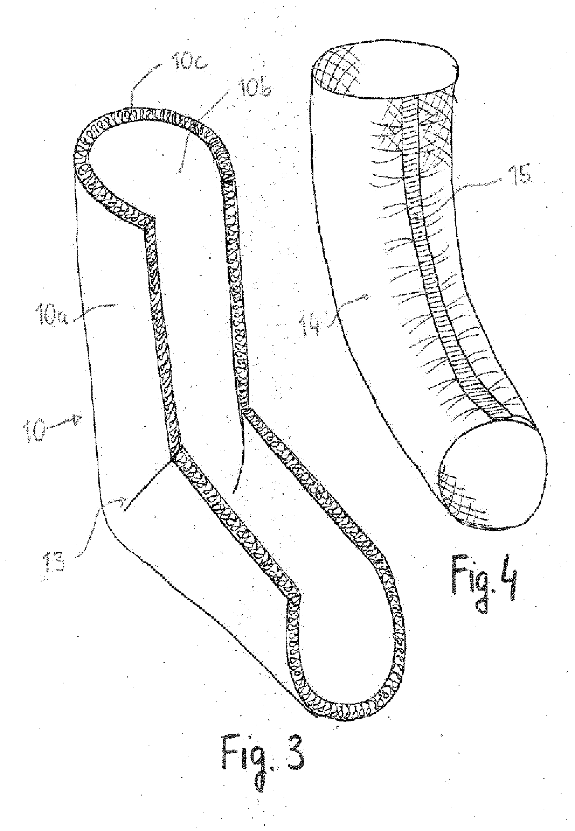

Figs. 3 and 4 show the 3·D-spacer fabric 10, preconfigured in a non closed tubular shape prepared to receive inside the stockinette 14, and with the edges of the cut out portions 11, 12, already joined by stitching providing a bent of and elbowed portion. The stockinette 14 is arranged inside the tubular jacket or external sleeve 10, so that extending coaxially within thereof, and joined by sewing of the major edges of the external sleeve 10 along the elastic longitudinal portion 15, providing in this way an elastic adaptation of the one-piece tubular assembly 16 (see Fig. 5) surrounding the part of the member to be covered procuring a cushioning effect (3-D-spacer fabric 10) and a protection of the skin of the patient by the inner textile tubular stockinette 14.

In addition to the joining by means of the cited sewing of the major edges of the external sleeve 10 along the elastic longitudinal portion 15, a further union between both stockinette 14 and tubular jacket 10 can be provided by providing a seam of the mouth piece area of both elements 10, 14 when shaped as a tubes. The joining is performed preferably on an external peripheral zone of the tubular jacket 10 to avoid any incidence on the skin of the patient. Thereby it is ensured that the textile tubular stockinette 14 will remain attached to the inner wall of the tubular jacket 10, without forming wrinkled areas, and then providing a suitable protection of the patient skin.

Moreover both said 3-D spacer fabric of the tubular jacket 10 and the knitted fabric of the stockinette 14 are washable and breathable so that providing a reusable integral protector.

At least when the proposed integral protector is intended for a foot and heel pressure ulcers protection, the one-piece tubular assembly of the two joined elements 10 and 14 will have an end portion wider that the other end and a suitable mark will be included in the protector to indicate the area of insertion of the member to protect.

### EXPERIMENTAL TESTS

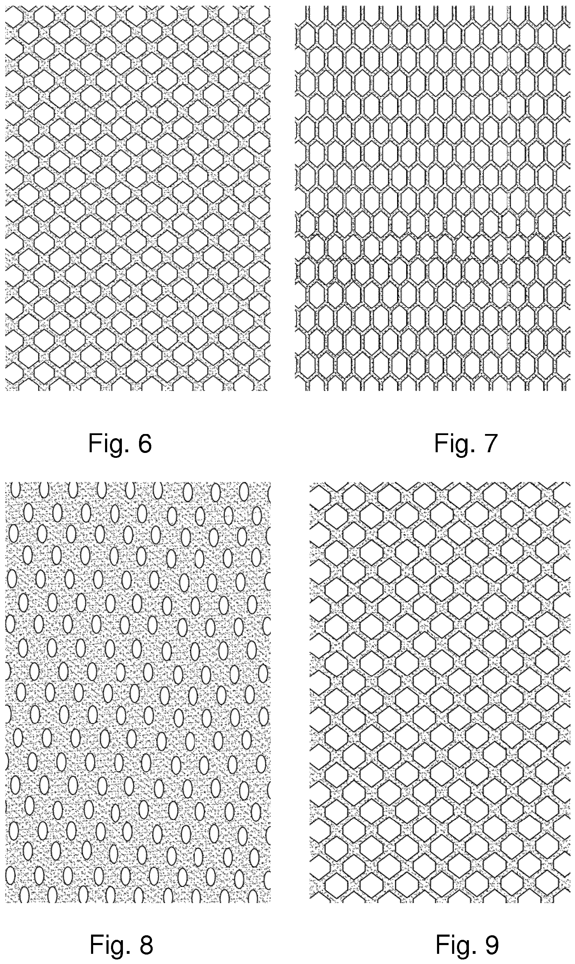

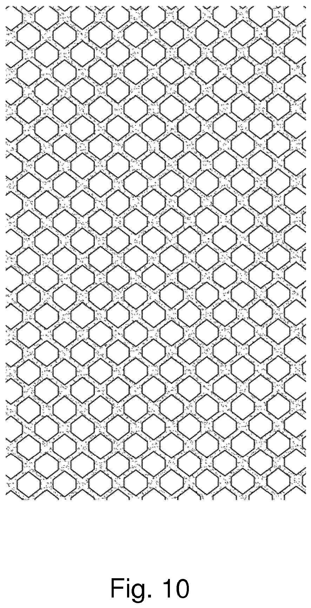

Five 3D spacer textiles known in the art (see corresponding pictures and technical details on Figs. 6 to 10) have been tested in order to finding a best 3D fabric for the production of the integral protector.

Two methods have been used to perform the test, a direct method using real patients and a dynamometric method using a series of weights (simulating a foot) programmed on a dynamometer.

The process consisted in marking the foot (real or artificial) with an iodine solution and then letting it rest during 4 seconds over a friseline sheet resting over the 3D fabric. This process is repeated 29 times on each one of the 3D spacer textiles initially tested (also without a textile) and in random order. A foot mark appears visible on the friseline sheet depending on the cushioning of the texted 3 D spacer.

## Claims

1. An integral protector for a living being member including a foot for prevention of pressure ulcers, comprising a one-piece tubular assembly (16) consisting of:
- a textile tubular stockinette (14) providing a tubular envelope intended to be in contact with the skin of the living being, said textile stockinette (14) comprising at least an elastic longitudinal portion (15) extending along a whole generatrix thereof, and
- a tubular jacket (10) made of a 3D spacer fabric providing an external sleeve, said tubular jacket receiving placed therein said textile stockinette (14), arranged coaxially and covering an inner surface thereof, said textile stockinette (14) being attached to said tubular jacket,
wherein said tubular jacket (10) operating as padding by decreasing the pressure acting on the cited living being member when in rest against a surface, said stockinette (14) acting as a skin protector and the one-piece tubular assembly providing a total protection of all the potential living member areas at risk against pressure ulcers.

2. Integral protector, according to claim 1, wherein said attachment between said stockinette (14) and said tubular jacket (10) is obtained at least by means of a seam of the mouthpiece area of both elements so that the integral protector remaining in place while the living member is moving.

3. Integral protector according to claim 2, wherein the cited seam of the mouthpiece is done on an external peripheral zone of the tubular jacket (10).

4. Integral protector according to any of the previous claims, wherein said textile stockinette is either knitted, woven or braided .

5. Integral protector, according to claim 1, o 3 wherein said tubular jacket (10) is obtained from a flat piece with two opposite cut-out portions (11, 12) by joining two opposite major edges of the flat piece, said cut-out portions (11, 12) when joined providing an angled portion corresponding to an elbow of the tubular jacket (10) suitable to accommodate against the external surface of a foot.

6. Integral protector, according to claim 1 or 3 wherein said tubular jacket (10) is obtained from a flat piece with two opposite cut-out portions (11, 12) by joining to said elastic longitudinal portion (15) of the stockinette (14), two opposite major edges of the flat piece, said cut-out portions (11, 12) providing an angled portion corresponding to an elbow of the tubular jacket (10) suitable to accommodate against the external surface of a foot.

7. Integral protector, according to claim 4 or 5, wherein said angled portion is obtained by means of a seam of the edges of said cut-out portions (11, 12) around a middle region of the tubular jacket (10) forming an angle with the axis thereof.

8. Integral protector, according to claim 1, wherein said elastic longitudinal portion (15) is provided while knitting said stockinette.

9. Integral protector, according to claim 1, wherein said elastic longitudinal portion (15) is a strip of regular width.

10. Integral protector, according to claim 1, wherein said tubular assembly (16) has an end portion wider than the opposite end.

11. Integral protector, according to claim 9, wherein one of the ends of the tubular assembly (16) includes a mark providing an indication of an introduction area of the member to protect.

12. Integral protector, according to claim 1, wherein the fabric of the textile stockinette (14) is washable and breathable.

13. Integral protector, according to claim 1, wherein the 3D spacer fabric of said tubular jacket (10) is washable and breathable.

## Patentansprüche

1. Integrale Schutzvorrichtung für ein Glied eines Lebewesens, einschließlich eines Fußes, für die Vorbeugung von Druckgeschwüren, welche eine einstückige rohrförmige Baugruppe (16) umfasst, bestehend aus:
- einem textilen rohrförmigen Trikot (14), welcher eine rohrförmige Umhüllung bereitstellt, welche dazu bestimmt ist, in Kontakt mit der Haut des Lebewesens zu sein, wobei der genannte textile Trikot (14) mindestens einen elastischen länglichen Teil (15) umfasst, welcher sich entlang einer ganzen Erzeugende desselben erstreckt, und
- einem rohrförmigen Mantel (10), hergestellt aus einem 3D-Abstandsgewirke unter Bereitstellung einer äußeren Hülse, wobei der genannte rohrförmige Mantel den genannten textilen Trikot (14) darin platziert empfängt, welcher koaxial angeordnet ist und eine Innenfläche desselben deckt, wobei der genannte textile Trikot (14) mit dem genannten rohrförmigen Mantel verbunden ist,
wobei der genannte rohrförmige Mantel (10) als Polsterung wirkt, indem es den Druck, welcher auf dem erwähnten Glied des Lebewesens agiert, verringert, wenn es an einer Oberfläche anliegt, wobei der genannte Trikot (14) als Hautschutzvorrichtung agiert und die einstückige rohrförmige Baugruppe einen gesamten Schutz gegen Druckgeschwüre aller möglicherweise gefährdeten Bereiche des Lebewesens bereitstellt.

2. Integrale Schutzvorrichtung nach Anspruch 1, wobei die genannte Verbindung zwischen dem genannten Trikot (14) und dem genannten rohrförmigen Mantel (10) mindestens mittels einer Naht des Mundstückbereichs beider Elemente erhalten wird, so dass die integrale Schutzvorrichtung an der Stelle bleibt, während sich das lebende Glied bewegt.

3. Integrale Schutzvorrichtung nach Anspruch 2, wobei die erwähnte Naht des Mundstücks auf einer äußeren Umfangszone des rohrförmigen Mantels (10) ausgeführt wird.

4. Integrale Schutzvorrichtung nach einem der vorhergehenden Ansprüche, wobei der genannte textile Trikot entweder gestrickt, gewebt oder geflochten ist.

5. Integrale Schutzvorrichtung nach Anspruch 1 oder 3, wobei der genannte rohrförmige Mantel (10) aus einem flachen Stück mit zwei entgegengesetzten ausgeschnittenen Teilen (11, 12) erhalten wird, indem zwei entgegengesetzte Hauptränder des flachen Stücks verbunden werden, wobei die genannten ausgeschnittenen Teile (11, 12), wenn sie verbunden sind, einen abgewinkelten Teil bereitstellen, welcher einer Abkröpfung des rohrförmigen Mantels (10) entspricht, welche dazu geeignet ist, sich an die Außenfläche eines Fußes anzupassen.

6. Integrale Schutzvorrichtung nach Anspruch 1 oder 3, wobei der genannte rohrförmige Mantel (10) aus einem flachen Stück mit zwei entgegengesetzten ausgeschnittenen Teilen (11, 12) erhalten wird, in dem zwei entgegengesetzten Hauptränder des flachen Stücks mit dem genannten elastischen länglichen Teil (15) des Trikots (14) verbunden werden, wobei die genannten ausgeschnittenen Teilen (11, 12) einen abgewinkelten Teil bereitstellen, welcher einer Abkröpfung des rohrförmigen Mantels (10) entspricht, welche dazu geeignet ist, sich an die Außenfläche eines Fußes anzupassen.

7. Integrale Schutzvorrichtung nach Anspruch 4 oder 5, wobei der genannte abgewinkelte Teil mittels einer Naht der Ränder der genannten ausgeschnittenen Teile (11, 12) um einen zentralen Bereich des rohrförmigen Mantels (10) herum, unter Bildung eines Winkels mit der Achse desselben, erhalten wird.

8. Integrale Schutzvorrichtung nach Anspruch 1, wobei der genannte elastische längliche Teil (15) während des Strickens des genannten Trikots bereitgestellt wird.

9. Integrale Schutzvorrichtung nach Anspruch 1, wobei der genannte elastische längliche Teil (15) ein Band regelmäßiger Breite ist.

10. Integrale Schutzvorrichtung nach Anspruch 1, wobei die genannte rohrförmige Baugruppe (16) einen Endteil aufweist, welcher breiter als das entgegengesetzte Ende ist.

11. Integrale Schutzvorrichtung nach Anspruch 9, wobei eins der Enden der rohrförmigen Baugruppe (16) eine Markierung beinhaltet, welche einen Hinweis eines Einführungsbereichs des zu schützenden Gliedes bereitstellt.

12. Integrale Schutzvorrichtung nach Anspruch 1, wobei das Gewirke des textilen Trikots (14) waschbar und atmungsaktiv ist.

13. Integrale Schutzvorrichtung nach Anspruch 1, wobei das 3D-Abstandsgewirke des genannten rohrförmigen Mantels (10) waschbar und atmungsaktiv ist.

## Revendications

1. Un protecteur intégral pour le membre d'un être vivant comprenant un pied pour la prévention des escarres, comportant un ensemble tubulaire d'une pièce (16) consistant en :
- un manchon tubulaire textile (14) offrant une enveloppe tubulaire destinée à être en contact avec la peau de l"être vivant, ce manchon textile (14) comportant au moins une portion longitudinale élastique (15) s'étendant le long de toute sa génératrice, et
- une gaine tubulaire (10) faite en tissu d'espacement 3D offrant une manche externe, cette gaine tubulaire recevant, placée à son intérieur ce manchon textile (14), aménagé coaxial et couvrant une surface intérieure de celui-ci, ce manchon textile (14), étant relié à cette gaine tubulaire,
dans lequel cette gaine tubulaire (10) agissant comme rembourrage en réduisant la pression agissant sur ce membre d'être vivant lorsqu'il est en repos contre une surface, ce manchon (14) agissant comme protecteur de la peau et l'ensemble tubulaire d'une pièce offrant une protection totale de toutes les régions du membre vivant potentiel risquant avoir des escarres.

2. Protecteur intégral, conformément à la revendication 1, dans lequel cette liaison entre ce manchon (14) et cette gaine tubulaire (10) est obtenue au moins au moyen d'une couture de la région d'ajutage des deux éléments de sorte que le protecteur intégral reste en place pendant que le membre vivant bouge.

3. Protecteur intégral, conformément à la revendication 2, dans lequel cette couture de l'ajutage est faite sur une région périphérique externe de la gaine annulaire (10).

4. Protecteur intégral, conformément à une quelconque des revendications précédentes, dans lequel ce manchon textile est tricoté, tissu ou tressé.

5. Protecteur intégral, conformément à la revendication 1 ou 3, dans lequel cette gaine annulaire (10) est obtenue d'une pièce plate ayant deux portions découpées opposées (11,12) en joignant deux bords plus grands opposés de la pièce plate, ces portions découpées (11,12) lorsqu'elles sont jointes offrant une portion en angle correspondant à un coude de la gaine tubulaire (10) adapté pour s'adapter contre la surface externe d'un pied.

6. Protecteur intégral, conformément à la revendication 1 ou 3, dans lequel cette gaine annulaire (10) est obtenue à partir d'une pièce plate ayant deux portions découpées opposées (11,12) en joignant cette portion longitudinale élastique (15) du manchon (14), deux bords plus grands opposés de la pièce plate, ces portions découpées (11, 12) offrant une portion en angle correspondant à un coude de la gaine tubulaire (10) adaptée pour s'adapter contre la surface externe d"un pied.

7. Protecteur intégral, conformément à la revendication 4 ou 5, dans lequel cette portion en angle est obtenue au moyen d'une couture des bordes de ces portions découpées (11,12) autour d'une région moyenne de la gaine tubulaire (10) formant un angle avec son axe.

8. Protecteur intégral, conformément à la revendication 1, dans lequel cette portion longitudinale élastique (15) est formée pendant le tricotage de ce manchon.

9. Protecteur intégral, conformément à la revendication 1, dans lequel cette portion longitudinale élastique (15) est une bande ayant une largeur régulière.

10. Protecteur intégral, conformément à la revendication 1, dans lequel cet ensemble tubulaire (16) possède une portion d'extrémité plus large que l'extrémité opposée.

11. Protecteur intégral, conformément à la revendication 9, dans lequel une de ces extrémités de l'ensemble tubulaire (16) comprend une marque offrant une indication d'une région d'introduction du membre à protéger.

12. Protecteur intégral, conformément à la revendication 1, dans lequel ce tissu de ce manchon textile (14) est lavable et respirant.

13. Protecteur intégral, conformément à la revendication 1, dans lequel ce tissu d'espacement 3D de cette gaine tubulaire (10) est lavable et respirant.
